# EUROPEAN PATENT SPECIFICATION

(11) **EP 4 234 069 B1**
(45) Date of publication and mention of the grant of the patent: **16.04.2025**
(21) Application number: 22209759.4
(22) Date of filing: 26.11.2022
(51) Int. Cl.: B01D 61/42, C02F 1/469, C02F 101/38, C02F 103/20, C02F 1/52

(54) **METHOD OF SEPARATION OF URIC ACID FROM POULTRY LITTER OR POULTRY MANURE**
VERFAHREN ZUR TRENNUNG VON HARNSÄURE AUS GEFLÜGELSTREU ODER GEFLÜGELMIST
PROCÉDÉ DE SÉPARATION D'ACIDE URIQUE À PARTIR DE LITIÈRE DE VOLAILLE OU DE FUMIER DE VOLAILLE

(30) Priority: 28.02.2022 PL 44049222
(43) Date of publication of application: 30.08.2023
(73) Proprietor: Uniwersytet Przyrodniczy w Poznaniu, 60-637 Poznan (PL)
(72) Inventor: LEWICKI, Andrzej, 62-081 Przezmierowo (PL); PROCHASKA, Krystyna, 62-002 Suchy Las (PL); SZCZYGIELDA, Mateusz, 62-052 Komorniki (PL); KUBIAK, Piotr, 61-408 Poznan (PL); CIESLIK, Marta, 62-002 Suchy Las (PL); DACH, Jacek, 28-300 Jedrzejów (PL)
(74) Representative: Augustyniak, Magdalena Anna

(56) References cited:
- CN-A- 107 055 712
- US-A- 4 196 290
- ROGERI DOUGLAS ANTONIO ET AL: "Composition of Poultry Litter in Southern Brazil", REVISTA BRASILEIRA DE CIÊNCIA DO SOLO, vol. 40, no. 0, 30 June 2016 (2016-06-30), pages 1 - 7, XP093054337, DOI: 10.1590/18069657rbcs20140697
- CROUCH NICHOLAS M A ET AL: "A re-evaluation of the chemical composition of avian urinary excreta", JOURNAL OF ORNITHOLOGY, SPRINGER BERLIN HEIDELBERG, BERLIN/HEIDELBERG, vol. 161, no. 1, 5 August 2019 (2019-08-05), pages 17 - 24, XP036983752, ISSN: 2193-7192, [retrieved on 20190805], DOI: 10.1007/S10336-019-01692-5

## Description

The object of the invention is the method of separation of uric acid from an aqueous solution of poultry litter or poultry manure using the technique of electrodialysis with an anion-selective membrane.

Uric acid is an organic chemical compound and a purine derivative represented by the formula C₅H₄N₄O₃. It is found in the environment both in the form of urates and in the form of uric acid crystals and uric acid hydrates. Due to their efficient water metabolism, uricotelic animals - birds, most reptiles, some insects, and terrestrial snails, excrete 70-90% of excess nitrogen from their bodies in the form of uric acid. The same is true of factoryfarmed poultry. The production process involves the generation of significant amounts of excrement - poultry litter, which is difficult to dispose of and which constitutes hazardous waste. The present application relates to the separation of uric acid from poultry litter with the aim of reducing nitrogen content in the litter to enable its methane monosubstrate fermentation and the use of separated uric acid crystals for manurial purposes.

Uric acid is characterised by variable solubility, depending on the pH, while its solubility increases in a curvilinear manner with the increase of pH of the solution in which it is present.

Known methods of uric acid separation mainly concern the separation of the substance in the process of laboratory sampling involving the use of chromatographs for medical research purposes. The low per-unit efficiency and the cost of these processes do not allow for their industrial use. At the same time, the uric acid salts formed by uric acid under poultry farming conditions, characterised by varying solubility, depending on the elements they are made up of, make it difficult to use a single, universal method of separation to obtain manurial substances.

Membrane methods can potentially be used for the separation of uric acid. They enable the separation of particle-sized contaminants and of molecules at the molecular or ionic levels. These processes are relatively new and have been developing rapidly in recent years. Advances in research in the development of membrane techniques make their use in environmental protection technically feasible and economically beneficial. Membrane separation processes and membrane reactors are techniques that have a wide range of uses today. Integrating membrane operations with traditional technologies or designing new production cycles based on membrane techniques is becoming an attractive field of engineering research. Nowadays, both polymeric and inorganic membranes with high selectivity and efficiency and with high thermal, chemical, and mechanical resistance, are being used more and more often for seawater desalination, wastewater treatment, recovery of valuable ingredients from wastewater, and for the separation of mixtures of organic compounds.

A common feature of all membrane techniques is the fact that the separation process takes place in a reactor divided by a membrane, which should be understood as a phase separating two other phases, and which acts as a passive or an active barrier to the transport of mass between them.

Another definition of a membrane indicates that it is a barrier that enables the transport of substances of different particle/molecule sizes between separated environments.

The transport through a membrane occurs due to the difference in chemical potentials Δµ on both sides of the membrane. The difference (Δµ) may be the result of a difference in pressure (ΔP), concentration (ΔC), temperature (ΔT), or electrical potential (ΔE).

US4196290A describes a method for treating chicken waste in which uric acid is separated by precipitation. CN107055712A describes a method for treating chicken manure by electrodialysis.

The present invention describes the method of separation of uric acid from poultry litter using an electrodialytic system. Its use makes it possible to achieve a significant variation of pH between the two sides of the membrane, which allows for bringing uric acid first into a soluble form, in an alkaline environment, and then causing its precipitation following the passage through an ion-selective membrane, in an acidic environment.

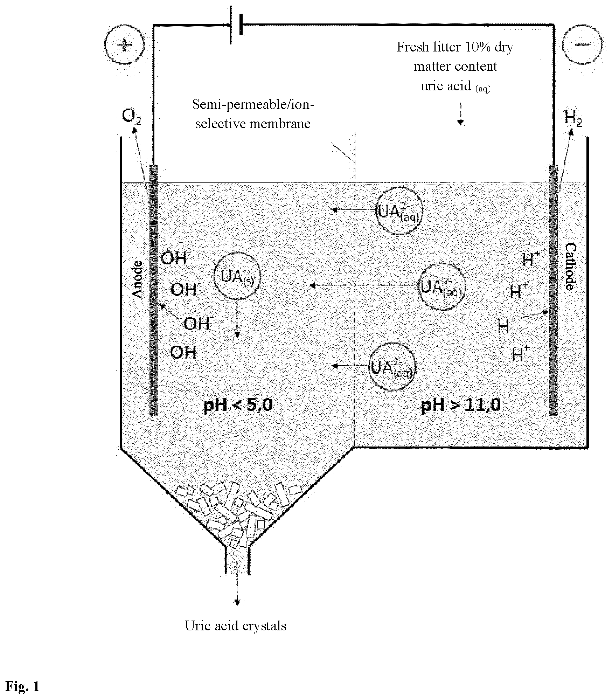

The proposed method involves feeding fresh litter into a strongly alkaline cathodic compartment, while allowing uric acid to transition to a highly ionised, soluble dinegative form (UA²⁻_{(aq)}), and then to migrate in the direction of a positively charged anode to an acidic anodic environment, in which the uric acid precipitates in a crystalline form (UA₍ₛ₎). This makes it possible to obtain uric acid in a collection funnel (Fig. 1).

The method according to the invention consists in placing an aqueous suspension of fresh poultry litter with a dry matter content ranging between 1 and 30%, in the cathodic compartment of an electrodialytic reactor. The anodic compartment is filled with water or with an aqueous solution of salts or bases. A semi-permeable, ion-selective, preferably anion-selective, membrane is first fitted between the compartments. Then, an electric current is supplied to electrodes placed in the electrodialyser, and the process is carried out at a current density in the range of 150 - 300 A/m². As a result of the flow of ions between the compartments, the pH in the two compartments is differentiated. The pH in the cathodic compartment rises, increasing the solubility of uric acid. Then, the urate radical, which has a negative charge, moves towards the anode, passing through the semi-permeable membrane. After the passage, it encounters the acidic environment of the anodic compartment, where the solubility of uric acid drops dramatically. This results in its precipitation in the form of crystals/hydrated flocs. Uric acid subsequently separates from the anodic compartment by sedimentation. Meanwhile, litter with significantly reduced nitrogen content is left in the cathodic compartment.

In another variant of the invention, an aqueous suspension of fresh poultry manure with a dry matter content in the range of 1-30% is placed in the cathodic compartment of the electrodialytic reactor. The anodic compartment is filled with an aqueous solution of a salt selected from among NaCl, Na₂SO₄, or a base (NaOH). An ion-selective - anion-selective semi-permeable membrane is first fitted between the compartments. Then, an electric current is supplied to electrodes and the process is carried out at a current density in the range of 150 - 300 A/m². As a result of the flow of ions between the compartments, the pH in the two compartments is differentiated. The pH in the cathodic compartment rises, increasing the solubility of uric acid. Then, the urate radical, which has a negative charge, moves towards the anode, passing through the semi-permeable membrane. After the passage, it encounters the acidic environment of the anodic compartment, where the solubility of uric acid drops dramatically. This results in its precipitation in the form of crystals/hydrated flocs. Uric acid subsequently separates from the anodic compartment by sedimentation. Meanwhile, poultry manure with significantly reduced nitrogen content is left in the cathodic compartment.

In the course of the conducted research, experiments were carried out using different reagents in the cathodic and anodic compartments, with the aim of creating an appropriate environment and ionic strength.

The results obtained clearly demonstrated the migration of uric acid from the diluate to the concentrate within 30 minutes of the duration of the experiments. In addition to that, flocculation of uric acid was observed on the side of the concentrate, which began to crystallise over time. The energy demand was also determined in the model systems (at the charge density of 161.6 A/m²), which amounted to 20.53 (Experiment 2) and 33.51 (Experiment 3) kWh/kg of uric acid.

The inputs that were obtained are therefore relatively high, however, it should be noted that the system that was used was not optimised, and the experiments were only intended to confirm the applicability of the method for the separation of uric acid from the litter. Making the calculation under such conditions, assuming that the price of electricity in the tariff for medium enterprises is about PLN0.42/kWh, the cost of electricity needed to obtain 1 kg of uric acid would be contained in the range of PLN9-PLN14. The volumetric flow rate per unit for uric acid amounted to 0.127 and 0.140 kg per m² of membrane per hour.

The method according to the invention has been illustrated in embodiment examples and in the drawing, in which Fig. 1 shows a conceptual diagram of uric acid precipitation using the process of electrodialysis.

### Example 1.

The method according to the invention involves placing an aqueous suspension of fresh poultry litter with a dry matter content in the range of 1-30% in the cathodic compartment of the electrodialyser. The anodic compartment is filled with water. A semi-permeable, ion-selective, preferably anion-selective, membrane is first fitted between the compartments. Then, an electric current is supplied to electrodes placed in the reactor and the process is carried out at a current density in the range of 150 - 300 A/m². As a result of the flow of ions between the compartments, the pH in the two compartments is differentiated. The pH in the cathodic compartment rises, increasing the solubility of uric acid. Then, the urate radical, which has a negative charge, moves towards the anode, passing through the semi-permeable membrane. After the passage, it encounters the acidic environment of the anodic compartment, where the solubility of uric acid drops dramatically. This results in its precipitation in the form of crystals/hydrated flocs. Uric acid subsequently separates from the anodic compartment by sedimentation. Meanwhile, litter with significantly reduced nitrogen content is left in the cathodic compartment.

### Example 2.

An aqueous suspension of fresh poultry manure with a dry matter content in the range of 1-30% is placed in the cathodic compartment of the electrodialyser. The anodic compartment is filled with an aqueous solution of NaCl salt. An ion-selective - anion-selective semi-permeable membrane is first fitted between the compartments. Then, an electric current is supplied to electrodes and the process is carried out at a current density in the range of 150 - 300 A/m². As a result of the flow of ions between the compartments, the pH in the two compartments is differentiated. The pH in the cathodic compartment rises, increasing the solubility of uric acid. Then, the urate radical, which has a negative charge, moves towards the anode, passing through the semi-permeable membrane. After the passage, it encounters the acidic environment of the anodic compartment, where the solubility of uric acid drops dramatically. This results in its precipitation in the form of crystals/hydrated flocs. Uric acid subsequently separates from the anodic compartment by sedimentation. Meanwhile, poultry manure with significantly reduced nitrogen content is left in the cathodic compartment.

### Example 3.

An aqueous suspension of fresh poultry manure with a dry matter content in the range of 1-30% is placed in the cathodic compartment of the electrodialyser. The anodic compartment is filled with an aqueous solution of NaSO₄ salt. An ion-selective - anion-selective semi-permeable membrane is first fitted between the compartments. Then, an electric current is supplied and the process is carried out at a current density in the range of 150 - 300 A/m². As a result of the flow of ions between the compartments, the pH in the two compartments is differentiated. The pH in the cathodic compartment rises, increasing the solubility of uric acid. Then, the urate radical, which has a negative charge, moves towards the anode, passing through the semi-permeable membrane. After the passage, it encounters the acidic environment of the anodic compartment, where the solubility of uric acid drops dramatically. This results in its precipitation in the form of crystals/hydrated flocs. Uric acid subsequently separates from the anodic compartment by sedimentation. Meanwhile, poultry manure with significantly reduced nitrogen content is left in the cathodic compartment.

### Example 4.

An aqueous suspension of fresh poultry manure with a dry matter content in the range of 1-30% is placed in the cathodic compartment of the electrodialyser. The anodic compartment is filled with an aqueous solution of NaOH salt. An ion-selective - anion-selective semi-permeable membrane is first fitted between the compartments. Then, an electric current is supplied and the process is carried out at a current density in the range of 150 - 300 A/m². As a result of the flow of ions between the compartments, the pH in the two compartments is differentiated. The pH in the cathodic compartment rises, increasing the solubility of uric acid. Then, the urate radical, which has a negative charge, moves towards the anode, passing through the semi-permeable membrane. After the passage, it encounters the acidic environment of the anodic compartment, where the solubility of uric acid drops dramatically. This results in its precipitation in the form of crystals/hydrated flocs. Uric acid subsequently separates from the anodic compartment by sedimentation. Meanwhile, poultry manure with significantly reduced nitrogen content is left in the cathodic compartment.

## Claims

1. A method of separation of uric acid from an aqueous solution of poultry litter or poultry manure, **characterised in that** in an electrodialytic reactor with compartments separated by a semi-permeable anion-selective membrane, an aqueous suspension of fresh poultry litter or an aqueous solution of fresh poultry manure with a dry matter content in the range of 1-30% is placed in the cathodic compartment of the reactor; the anodic compartment is filled with water or with an aqueous solution of salts or bases when poultry litter is used; when poultry manure is used, the anodic compartment is filled with an aqueous solution of a salt or a base; and then, electric current is supplied to electrodes placed in the compartments and the process is carried out at a current density in the range of 150 - 300 A/m²;
wherein uric acid is brought into a soluble dinegative ionised urate form in the strongly alkaline environment of the cathodic compartment, allowing it to pass through the membrane in the acidic environment of the anodic compartment, after which, uric acid crystals are precipitated from the acidic environment of the anodic compartment; after that said uric acid crystals are separated by sedimentation, while nitrogen content in the cathodic compartment is reduced.

2. The method according to claim 1, **characterised in that** the aqueous solution of a salt is the solution of NaCl.

3. The method according to claim 1, **characterised in that** the aqueous solution of a salt is the solution of NaSO₄.

4. The method according to claim 1, **characterised in that** the aqueous solution of a salt is the solution of NaOH.

## Patentansprüche

1. Ein Verfahren zur Abscheidung der Harnsäure aus einer wässrigen Lösung von Geflügeleinstreu oder Geflügel dung, **dadurch gekennzeichnet, dass** eine wässrige Suspension aus frischer Geflügeleinstreu oder eine wässrige Lösung aus frischem Geflügeldung mit Trockenmassegehalt im Bereich von 1 - 30% in die kathodische Kammer des Reaktors eines elektrodialytischen Reaktors, deren Kammern mit einer semipermeablen anionenselektiven Membran getrennt sind, gefüllt wird; die anodische Kammer wird dann mit Wasser oder mit einer wässrigen Lösung eines Salzes oder einer Base gefüllt, wenn die Geflügeleinstreu behandelt wird; wenn der Geflügeldung behandelt wird, wird die anodische Kammer mit einer wässrigen Lösung eines Salzes oder einer Base gefüllt; dann werden die Elektroden, die in den Kammern angeordnet sind, mit Strom versorgt und das Verfahren wird mit einer Stromdichte im Bereich von 150 - 300 A/m² durchgeführt;
wobei die Harnsäure in der stark alkalischen Umgebung der kathodischen Kammer in ein lösliches, zweifach negativ ionisiertes Harnsalz umgewandelt wird, wodurch sie durch die Membran in die saure Umgebung der anodischen Kammer gelangen kann, woraufhin die Harnsäurekristalle aus der sauren Umgebung der anodischen Kammer ausgeschieden werden; danach werden die Harnsäurekristalle durch Sedimentation getrennt, wobei der Stickstoffgehalt in der kathodischen Kammer reduziert wird.

2. Verfahren nach Anspruch 1, **dadurch gekennzeichnet, dass** die wässrige Lösung eines Salzes die Lösung von NaCl ist.

3. Verfahren nach Anspruch 1, **dadurch gekennzeichnet, dass** die wässrige Lösung eines Salzes die Lösung von NaSO₄ ist.

4. Verfahren nach Anspruch 1, **dadurch gekennzeichnet, dass** die wässrige Lösung eines Salzes die Lösung von NaOH ist.

## Revendications

1. Procédé de séparation de l'acide urique d'une solution aqueuse de litière de volaille ou de fumier de volaille, **caractérisé en ce que**, dans un réacteur électrodialytique dont les compartiments sont séparés par une membrane semi-perméable sélective des anions, une suspension aqueuse de litière de volaille fraîche ou une solution aqueuse de fumier de volaille frais d'une teneur en matière sèche comprise entre 1 et 30% est placée dans le compartiment cathodique du réacteur ; le compartiment anodique est rempli d'eau ou d'une solution aqueuse de sels et de bases lorsque de la litière de volaille est utilisée ; lorsque du fumier de volaille est utilisé, le compartiment anodique est rempli d'une solution aqueuse d'un sel ou d'une base ; ensuite, un courant électrique est fourni aux électrodes placées dans les compartiments et le processus est effectué à une densité de courant comprise entre 150 et 300 A/m² ;
dans lequel l'acide urique est transformé en une forme soluble d'urate ionisé dans l'environnement fortement alcalin du compartiment cathodique, ce qui lui permet de traverser la membrane dans l'environnement acide du compartiment anodique, après quoi des cristaux d'acide urique sont précipités dans l'environnement acide du compartiment anodique ; ces cristaux d'acide urique sont ensuite séparés par sédimentation, tandis que la teneur en azote dans le compartiment cathodique est réduite.

2. Procédé selon la revendication 1, **caractérisé par le fait que** la solution aqueuse d'un sel est la solution de NaCl.

3. Procédé selon la revendication 1, **caractérisé par le fait que** la solution aqueuse d'un sel est la solution de NaSO₄.

4. Procédé selon la revendication 1, **caractérisé par le fait que** la solution aqueuse d'un sel est la solution de NaOH.
